# EUROPEAN PATENT APPLICATION

(11) **EP 4 529 829 A1**
(43) Date of publication of application: **02.04.2025**
(21) Application number: 24203364.5
(22) Date of filing: 27.09.2024
(51) Int. Cl.: A61B 3/14, A61B 5/00

(54) **PORTABLE MODULAR SYSTEM FOR DIAGNOSTIC INSTRUMENTS**

(30) Priority: 29.09.2023 IT 202300020169
(71) Applicant: Espansione Marketing S.p.A., 40050 Funo di Argelato (BO) (IT)
(72) Inventor: FIER, Alessandro, 40136 Bologna (IT)
(74) Representative: Manzella & Associati

(57) **Abstract**

The portable modular system for diagnostic instruments comprises a first module (2) comprising at least one image detection and acquisition device (3) and a second module (4) comprising a control unit (5) configured to receive and process the images acquired by said image detection and acquisition device (3), a support member (6) configured to removably support said first module (2) and said second module (4) in respective positions.

## Description

### Technical field

The present invention relates to a portable modular system for diagnostic instruments.

### Prior art

The use of special medical instruments to perform diagnostic procedures is known.

In particular, equipment capable of diagnosing pathologies which affect, for example, the eyelids, causing chronic inflammatory states of the periorbital region, are known. Such pathologies are manifested, for example, by an alteration of tear production, which is excessive, insufficient or in any case qualitatively altered, as in the case of dry eye syndrome. The known pathologies which can manifest such symptoms are, for example, blepharitis, chalazion, stye and meibomitis.

To make a diagnosis of such dysfunctions, diagnostic instruments usually used in specialized medical facilities or hospital facilities are known. Such instruments are able to detect images of the parts in question, to appropriately enlarge them, so that they can be analysed by the specialist.

It is now well established that with an early diagnosis of these pathologies, as indeed in general, it is possible to greatly increase the possibility of healing, the effectiveness as well as the speed of action of the available therapeutic methods.

By way of example, in the field of dermatology and ophthalmic medicine, the aforementioned specialized medical facilities have very complex, therefore expensive diagnostic instruments, provided with a camera for the acquisition of images of the parts of the body under examination, which allow specialists to appreciate the symptomatic state of the patient in depth, so as to safely elaborate an early diagnosis.

It often occurs that only on the occasion of specialist checks is it possible to obtain a diagnosis of the pathology, thus increasing the risk of a late diagnosis, when the pathology, in an advanced stage of development, manifests obvious symptoms. Therefore, systems have been developed which allow to solve the aforesaid problem.

An example of the mentioned systems is illustrated in European Patent No. EP3755206 on behalf of the Applicant. The patent discloses a portable modular system for diagnostic instruments comprising at least one device for detecting and acquiring images, of the type of a video camera or a camera, and an electronic data processing unit configured to receive and process the images acquired by the device. The electronic unit and the device are removably mounted on a support member in respective positions. The support member is made by means of an L-shaped bracket, which bears a first seat for fixing the device and a second seat for fixing the electronic unit. The support member is connected to a coupling member to allow the coupling to a base body or a handle.

The system described above is deemed a considerable improvement in the field as it is easily installable in any environment, whether it is a specialized medical centre or hospital facility or can be used manually at a paramedical centre or a pharmacy and allows a diagnostic procedure to be easily performed.

Despite this, a problem complained of is that the known system is still not sufficiently manageable for an operator assigned to perform diagnostic procedures, since it has dimensions which hinder the operator during the execution of such procedures, especially where manual use of the system is necessary.

A further problem complained of is that, if more than one examination must be performed on a patient, the operator, at the end of a first examination, must operate the processing unit and the detection device again to start the new diagnostic procedure. This is time-consuming.

Therefore, the need is felt to identify new solutions to the problems identified.

### Disclosure

The object of the present invention is that of solving the above-mentioned problems, devising a portable modular system which allows to perform diagnostic procedures simply and effectively.

Within this task, it is a further object of the present invention to provide a system which allows to decrease the time needed to carry out an excellent diagnosis for each patient.

A further object of the invention is to devise a system which is ergonomic.

The cited objects are achieved, according to the present invention, by the portable modular system for diagnostic instruments according to claim 1, as well as by the computer program according to claim 9.

The portable modular system for diagnostic instruments comprises a first module comprising at least one image detection and acquisition device, for example a camera or a video camera, and a second module comprising a control unit configured to receive and process the images acquired by said image detection and acquisition device, said control unit being provided with a user interface; a support member configured to removably support said first module and said second module in respective positions.

The control unit comprises an electronic computer and a memory readable by said electronic computer.

The memory comprises instructions which, when performed by said electronic computer, cause the computer to perform the step of acquiring information related to a new patient or related to a patient already registered (a);
displaying, by means of said user interface, a list of examinations which can be performed by the system (b).

The next step envisages acquiring information about the examination or examinations to be performed by said patient (c) and preferably to display, by means of said user interface, information about the emission of a light at a predetermined wavelength to illuminate at least one eye of said patient (d).

The next step envisages acquiring at least one image of at least one eye of the patient (e).

The next step is to save said at least one image and process it by means of said control unit (f).

The aforesaid steps d-f are repeated until all the selected examinations are completed.

Preferably, the support member comprises a resting and/or gripping element having an elongated shape which extends, in a configuration of use, on a substantially vertical plane, and an operating portion, associated with one end of said resting and/or gripping element, which shapes a first seat adapted to house said first module, said resting and/or gripping element also shaping a second seat for said second module. The system has a compact structure thanks to the configuration of the resting and/or gripping element comprising both seats for the respective operating modules. The compact structure facilitates the operator in performing the diagnostic examinations.

Preferably, said resting and/or gripping element extends along a vertical direction, in use.

Advantageously, said operating portion defines a cavity therein which is adapted to define said first seat for said first module. This thereby further optimises the dimensions of the system.

Preferably, said operating portion is made by a hollow cylinder.

Preferably, said resting and/or gripping element has a substantially tubular shape.

Preferably, said resting and/or gripping element is made of a pair of half-casings coupled to each other, said first module being housed between said half-casings at said operating portion.

Advantageously, said resting and/or gripping element comprises a first portion, which extends along a substantially straight direction, and a second portion, connected to said first portion, which extends along an inclined direction with respect to the direction of said first portion, on the same lying plane of said first portion. In practice, said first portion and said second portion define a region having a predetermined radius of curvature adapted to facilitate gripping said resting and/or gripping element. The operator can therefore comfortably grasp the resting and/or gripping element thanks to its ergonomic configuration. This is advantageous both in the case where the system is manually supported by the operator and in the case where the system is associated with a base body since, in the second case, the operator can carry out the necessary operations with the two modules while maintaining a comfortable grip of the system.

Preferably, said resting and/or gripping element has a plurality of grooves made on at least a part of the outer surface, adapted to facilitate gripping by an operator.

Preferably, said grooves extend for at least a part of said first portion of said resting and/or gripping element and for at least a part of said second portion.

Preferably, said second seat is obtained from a recess.

Preferably, said recess extends along a longitudinal direction defining a groove.

Preferably, said second seat is made on said first portion.

Preferably, said second seat is obtained on a surface of said resting and/or gripping element which is opposite with respect to the surface facing the patient.

Preferably, said control unit of said second module is integrated in a portable tablet.

Preferably, said second module is provided with attachment means configured to ensure a removable connection to said second seat.

Preferably, said attachment means comprise an element which functions as an attachment base to the second module.

Preferably, said element extends longitudinally and has an elongated shape.

Preferably, said element is provided with an outwardly projecting portion adapted to be associated with said second seat.

Preferably, said projecting portion has a shape complementary to said second seat.

Preferably, said projecting portion has an elongated shape and extends along a longitudinal direction.

Preferably, said first module also comprises a lighting device, frontally associated with said device for the detection and processing of images.

Preferably, said lighting device comprises a plurality of light emitting diodes and an electrical circuit for controlling said light emitting diodes.

Preferably, said electrical control circuit is made as a printed circuit board.

Preferably, said system further comprises transmission means which allow data to be transmitted to a remote server.

Said transmission means preferably allow data to be transmitted via the internet.

Preferably, the transmitted data comprises the acquired images, data related to the patients and/or the examinations carried out and/or data related to the results of such examinations.

A further object of the present invention is a computer program comprising instructions which cause the system to perform the step of acquiring information related to a new patient or related to a patient already registered by means of said user interface of said control unit (a).

The next step envisages displaying, by means of said user interface, a list of examinations which can be performed by the system (b).

The program envisages acquiring information about the examination or examinations which said patient must perform (c).

Preferably, the program comprises the step of displaying, by means of said user interface, information on the emission of a light at a predetermined wavelength to illuminate at least one eye of said patient (d).

The program envisages acquiring at least one image of at least one eye of the patient (e).

The next step is to save said at least one image and process it by means of said control unit (f).

The aforesaid steps d-f are repeated until all the selected examinations are completed. Performing the examinations one after the other is advantageous because it reduces the overall time needed to carry out a diagnosis for each patient with an advantage for both the patient and the operator.

Furthermore, the computer program implemented by the system allows to effectively guide an operator on how to carry out each examination. In particular, the program acquires information about the patient and the examination or examinations to be carried out and, based on such information, displays on the user interface, where necessary, the wavelength of light which must be emitted to illuminate at least one eye of the patient, and acquires at least one image of the patient's eye and processes it appropriately.

Preferably, following the step of displaying the wavelength of the light to be emitted, the program includes the step of activating a lighting device, by means of the operator, to emit the light at the indicated wavelength. Alternatively, it is possible to envisage that the program proceeds to automatically activate the lighting device, by means of the control unit, so as to emit a light at a predetermined wavelength, which depends on the type of examination selected. Preferably, the list of examinations which can be performed by the system comprises: meibography, which is intended to assess the level of dysfunction of the meibomian glands, interferometry, which is intended to assess the thickness of the surface lipid layer located above the normal tear film in addition to the thickness of the fluid layer covering the anterior surface of a contact lens, an examination which is intended to detect the possible presence of Demodex mites by visual inspection of an image acquired at a high magnification, an examination called "Redness Efron grading" which assesses the percentage of redness and the severity of redness in the conjunctival area, an examination which is intended to provide information on the diameter of the pupil in an ambient light condition, an examination which is intended to investigate the amount of accumulated tears, in particular by investigating a lower portion of the patient's eye in which tears generally accumulate. It should be noted that the program allows numerous types of diagnostic examinations to be performed, therefore, it makes it possible to evaluate different aspects of a patient's eye with the same system.

Advantageously, the step of repeating the aforesaid steps d-f until the end of the performance of all the selected examinations is performed automatically.

Preferably, the aforesaid steps d-f are repeated in succession if said patient must carry out more than one examination.

Preferably, before the step of preferably displaying, by means of said user interface, information on the emission of a light at a predetermined wavelength to illuminate at least one eye of said patient, the program comprises the step of choosing the order of performance of the examinations.

Preferably, the step of acquiring information about the examination or examinations to be carried out envisages obtaining such information from the system memory and/or from a remote server, for example from a cloud server.

Preferably, said information on the examination or examinations to be carried out is displayed on the user interface.

Alternatively, the operator can enter, by means of the user interface, information regarding the examination or examinations which the patient must carry out and preferably save this information, on the system memory and/or on a remote server.

Advantageously, for at least one type of diagnostic examination, the program envisages, following step f, the step of displaying on said user interface a questionnaire associated with said predetermined diagnostic examination in progress and receiving further data about the patient. Thereby, it is possible to acquire further information about the patient which is used to carry out an assessment of the patient's disorder.

Preferably, the program comprises the step of displaying, by means of said user interface, information related to the use of a diffuser member associable with said first module. For example, in the case of interferometry examination, this step is performed.

Preferably, the step of processing at least one image by means of said control unit envisages changing the contrast and/or brightness and/or controlling the blurring of the image and/or carrying out three-dimensional processing.

The image processing step may also envisage identifying the pupil and measuring the diameter of the pupil, if an examination is selected for verification on a patient's pupil.

Preferably, the program envisages, before said step of acquiring information related to a new patient or a patient already registered, an activation step. In particular, the activation step envisages displaying information related to the entry of an activation code and receiving a datum corresponding to the activation code. The activation code datum is then compared with a stored datum and, if a match is found between the data, activation is allowed.

Preferably, said activation code datum is stored in a remote archive, e.g., an archive of a remote server.

Preferably, said remote server is a cloud server.

A further aspect of the present invention is a data transmission system comprising at least one portable modular system for diagnostic instruments comprising a first module comprising at least one image detection and acquisition device, for example a camera or a video camera, and a second module comprising a control unit provided with a user interface, said control unit being configured to receive and process data related to the images acquired by said image detection and acquisition device; a support member configured to removably support said first module and said second module in respective positions; transmission means for transmitting said data related to the images acquired to a remote server; said data transmission system also comprising a remote server comprising means for receiving data from said portable modular system.

Preferably, said control unit is also configured to process further data comprising information related to a new patient or related to a patient already registered and/or information about the examination or examinations said patient must perform and/or information about the outcome of the examination or examinations carried out by each patient.

Preferably, said transmission means of said portable modular system are adapted to transmit to a remote server said data related to a new patient or related to a patient already registered and/or information about the examination or examinations which said patient must perform and/or information about the result of the examination or examinations carried out by each patient.

Preferably, said remote server comprises relative transmission means for transmitting data to said portable modular system, said data comprising information on at least one activation code so as to allow activation of said at least one portable modular system.

Advantageously, said data to be transmitted to said portable modular system comprise information regarding the activation and/or deactivation of an examination from the examination list, information for setting predetermined parameters of the at least one image detection and acquisition device used in the examinations and on the calibration of the same device. Thereby, a configuration of the program installed on each portable modular system can be carried out remotely, in a differentiated manner for each customer.

Preferably, said predetermined parameters of said image detection and acquisition device comprise brightness and contrast.

Preferably, said data to be transmitted to said portable modular system also comprises information on enabling and/or disabling a file saving function of said modular system on a data archive of said server.

Preferably, said remote server comprises data processing means.

Preferably, said remote server is a cloud server.

Preferably, said data transmission system comprises a web API (Application Programming Interface) adapted to function as an interface between said modular system and said remote server.

### Description of the drawings

The details of the invention will become more evident from the detailed description of a preferred embodiment of the portable modular system according to the invention, illustrated by way of example in the accompanying drawings, wherein:
Figure 1 shows a perspective view of the system according to the present invention;

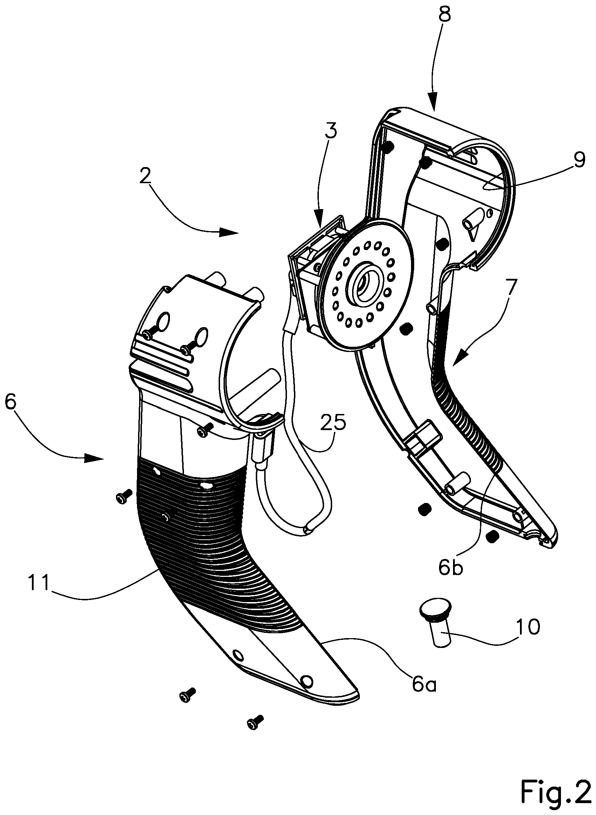

Figure 2 shows a perspective view of a detail of the system in a configuration where it is partially disassembled;
Figures 3, 4 and 5 respectively show an axonometric view, a rear front view and a front frontal view of the detail illustrated in Figure 2;
Figure 6 shows a perspective view in a disassembled configuration of a detail of the system;
Figure 7 shows an axonometric exploded view of a further detail of the system;
Figure 8 shows an exploded perspective view of a different detail of the system;
Figures 9 and 10 respectively show a front view and a perspective view of the detail illustrated in Figure 8;
Figure 11 shows a block diagram illustrating the steps implemented by the computer program object of the invention;
Figure 12 shows a data transmission system according to the present invention comprising a plurality of portable modular systems.

### Description of embodiments of the invention

With particular reference to such figures, the portable modular system for diagnostic instruments according to the invention, which will be indicated as system in the following disclosure for the sake of simplicity, is referred to as a whole by reference numeral 1.

The system 1 comprises a first module 2 comprising at least one image detection and acquisition device 3, which can be a camera or a video camera.

The system 1 further comprises a second module 4 comprising a control unit 5 provided with a user interface 23.

The control unit 5 is configured to receive and process the images acquired by the image detection and acquisition device 3. The control unit 5 is also configured to process further types of data, for example data related to each patient and/or data related to the examinations which each patient must carry out and/or data on the results of such examinations. In practice, the control unit 5 is configured to process data received by means of the user interface 23 as well as by means of the image detection and acquisition device 3.

The control unit 5 comprises an electronic computer and a memory readable by the electronic computer, both not visible in the figures.

The system 1 further comprises a support member 6 configured to removably support the first module 2 and the second module 4 in respective positions. The support member 6 allows the system to be coupled on a base body, not illustrated in the figures, or it can be manually supported by an operator assigned to perform the diagnostic procedures.

The support member 6 comprises a resting and/or gripping element 7 which extends, in a configuration of use, on a substantially vertical plane. More in particular, the resting and/or gripping element 7 extends along a vertical direction, in use.

Such an element 7 has an elongated shape and a substantially tubular structure.

At one end, the resting and/or gripping element 7 is associated with an operating portion 8 which shapes a first seat 9 adapted to house the first module 2. Preferably, the resting and/or gripping element 7 is integral with the operating portion 8. Even more preferably, the resting and/or gripping element 7 and the operating portion 8 are made in a single piece.

The operating portion 8 shapes a cavity therein constituting the first seat 9 for the first module 2. In particular, the operating portion 8 has a hollow cylinder shape which internally delimits the first seat 9 for the first module 2. The shape of the operating portion can certainly be different, for example spherical or prismatic.

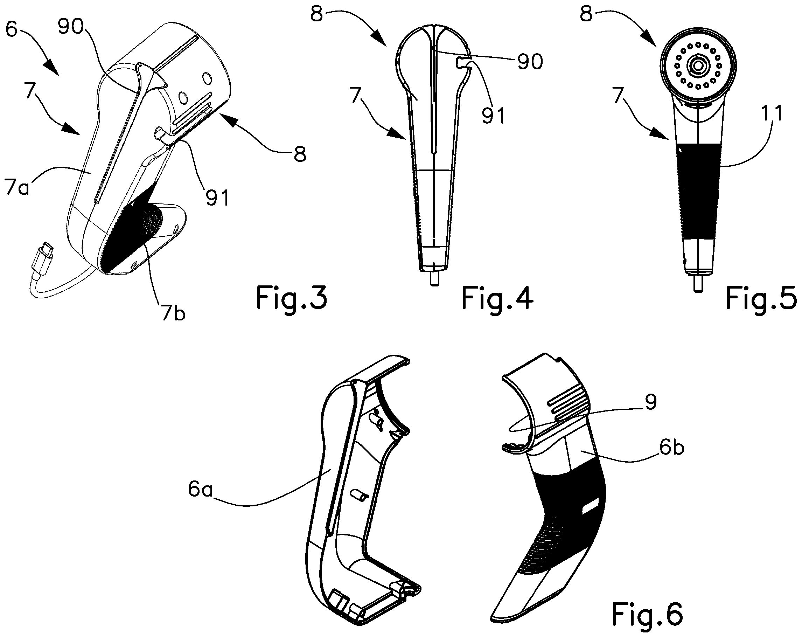

The resting and/or gripping element 7 shapes a second seat 90 arranged for the second module 4 (see Figures 3 and 4). Preferably, the second seat 90 is made by a recess adapted to receive attachment means associated with the second module 4, as will be disclosed below. The recess extends along a longitudinal direction, defining a groove.

The resting and/or gripping element 7 is provided with respective coupling means 10, for example of a coupling pin, for the connection with the base body. The coupling means 10 are preferably arranged at an end of the resting and/or gripping element 7 which is opposite with respect to the operating portion 8.

The resting and/or gripping element 7 comprises a first portion 7a extending along a straight direction and a second portion 7b, integral with the first portion 7a, extending along an inclined direction with respect to the direction of the first portion on the same lying plane as the first portion 7a. The first portion 7a is suitably connected to the second portion 7b and such portions define a region with a predetermined radius of curvature which facilitates an operator in gripping the resting and/or gripping element 7, especially in the case of manual use of the system 1. Therefore, the resting and/or gripping element 7 has an ergonomic configuration.

Advantageously, a series of grooves 11 are made on a part of the outer surface of the resting and/or gripping element 7, adapted to facilitate gripping the support member 6. The grooves 11 extend for at least a part of the first portion 7a and for at least a part of the second portion 7b.

Preferably, each groove 11 defines a sort of ring and the rings are made about the same distance from each other along the longitudinal extension of the resting and/or gripping element 7.

The second seat 90, arranged to be associated with the second module 4, is made on the first portion 7a. In particular, the second seat 90 is obtained on a rear surface of the first portion 7a of the resting and/or gripping element 7, i.e., on a surface which is opposite with respect to the surface facing the patient.

According to an embodiment, the support member 6 is made of a pair of half-casings 6a, 6b coupled to each other and the first module 2 is housed between the half-casings at the operating portion 8.

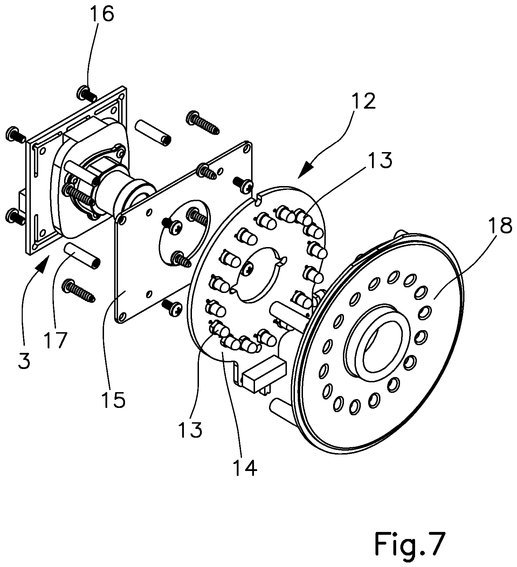

The first module 2 comprises, in addition to at least one image detection and acquisition device 3, a lighting device 12, frontally associated with the image detection and acquisition device 3 (see Figure 7).

The lighting device 12 is configured to emit electromagnetic radiation at different wavelengths, depending on the diagnostic examination which must be carried out. By way of example, the lighting device can emit an infrared light, a red light, a white light.

The lighting device 12 comprises a plurality of light emitting diodes 13 distributed on a support 14 and an electric control circuit of the light emitting diodes 13. Preferably, the electric control circuit is made as a printed circuit board which also functions as a support 14 for the light emitting diodes 13. The components of the electrical circuit are not disclosed, as known per se.

The image detection and acquisition device 3 is associated with a support element 15 by means of suitable connection means 16 and a series of spacer elements 17 are interposed between the support element 15 and the device 3 itself. The lighting device 12 is mounted in front of the support element 15 and a closing element 18 is mounted in front of the lighting device 12 (see Figure 7).

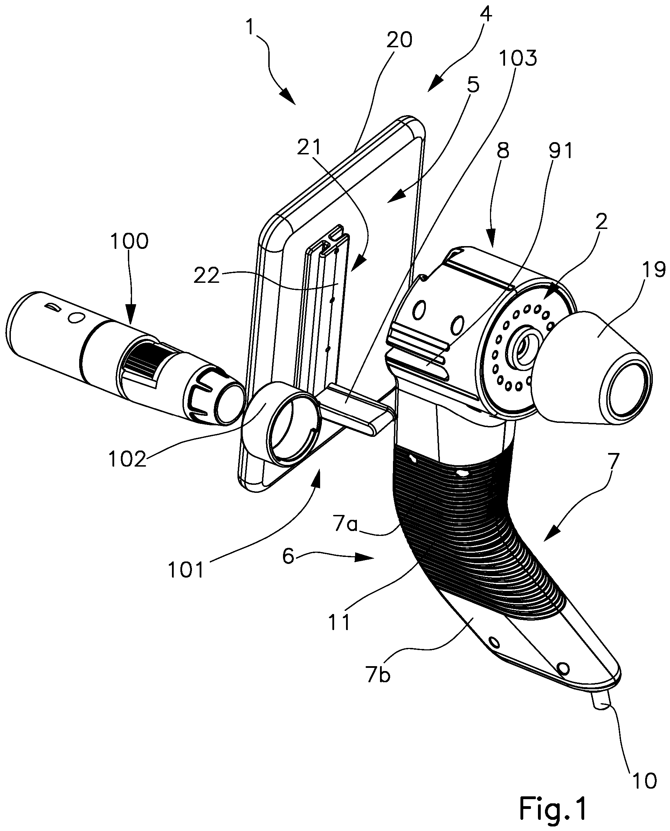

The first module 2 is associable with a diffuser member 19 which allows the flow of electromagnetic radiation emitted by the lighting device 12 to be distributed uniformly within a given solid angle. In more detail, the diffuser member 19 is adapted to be connected to the operating portion 8, in a frontal position with respect to the lighting device 12 (see Figure 1).

The image detection and acquisition device 3 and the control unit 5 are connected, for example by means of wires 25 or wirelessly, so that the images detected by the former can be processed, in real time, by the latter, for example to extract, record, store processed diagnostic data.

The control unit 5 of the second module 4 can advantageously be integrated in a portable tablet 20, thus easily transportable and usable (see Figure 1 and Figures 8-10).

The tablet 20 is provided with relative attachment means 21 for the removable connection to the second seat 90. The attachment means 21 can be made of an element having an elongated shape extending along a longitudinal direction, of a bracket type. Such an element is fixed to an outer surface of the tablet and functions as an attachment base to the tablet. The element is provided with an outwardly projecting portion 22 which is arranged to be inserted in the second seat 90. The projecting portion 22 has a shape complementary to the second seat 90 for coupling with the seat 90.

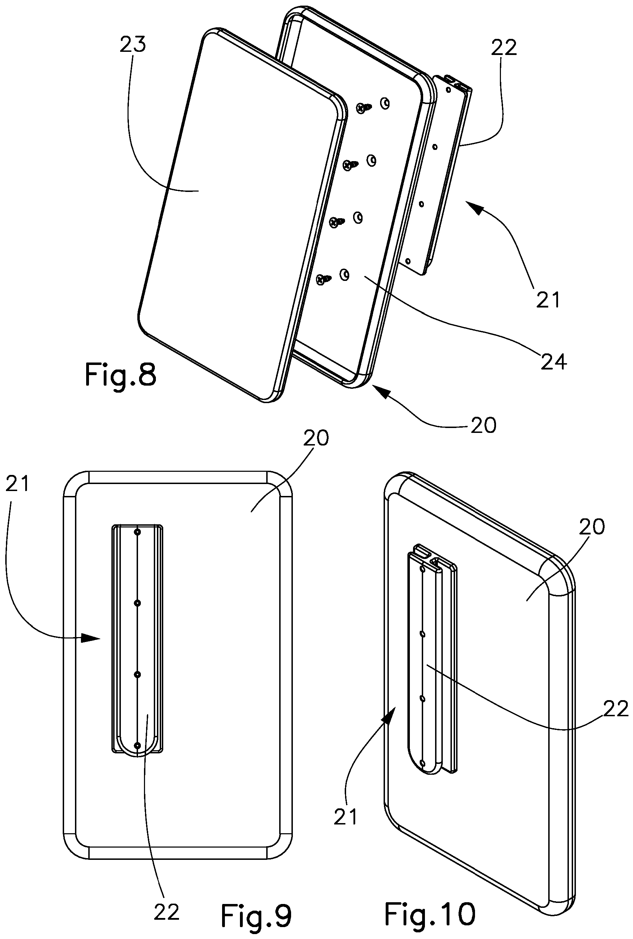

The tablet 20 is provided with a user interface 23, visible in Figure 8. The tablet 20 can be provided with a suitable protective case 24, as always illustrated in Figure 8.

According to an aspect of the invention, the system 1 also comprises a microscope 100, associated with the support member 6 by means of a connection member 101, which can be used to perform specific diagnostic procedures. The connection member 101 comprises a first element 102, made for example by a ring, adapted to be associated with the microscope 100 to keep it in a stable position, in a phase of use, and a second element 103 connected to the first element 102. The second element 103 is associable with a third seat 91 obtained on the support member 6. The seat 91 is made at an outer surface of the operating portion 8. The third seat 91 preferably has a shape complementary to the second element 103.

The microscope is in communication, preferably wireless, with the control unit 5 to transmit the acquired data which must be suitably processed by the control unit 5. The microscope can comprise a relative image acquisition device, not illustrated in the Figures, which communicates wirelessly with the control unit 5.

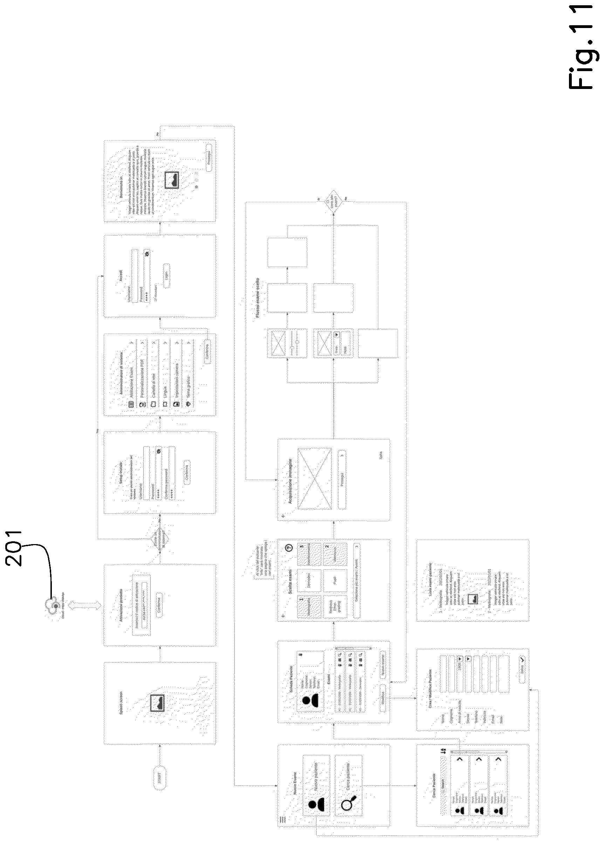

The modular system 1 comprises transmission means, not depicted in the figures, which allow data to be transmitted to a remote server 200, as will be better explained below. The transmission means allow data to be transmitted via the internet. The data comprises the acquired images, data related to the patients and/or the examinations carried out and/or data related to the results of such examinations. A further object of the present invention is a computer program implemented by the system 1 (see Figure 11).

The program comprises instructions causing the system 1 to first perform an activation step which envisages displaying information related to the entry of an activation code and receiving a datum corresponding to the activation code. The activation code datum is compared with a datum stored in a remote archive, such as a cloud server archive, and if a match is found between the data, activation is allowed.

In the case of the first start-up of the program, the program comprises then a step of creating an administrator user. In particular, the program envisages receiving data related to a username and an administrator password.

Subsequently, the program envisages a login step which comprises receiving data related to a username and a password. As an alternative to receiving such data, it is possible to envisage an access token which allows to connect to the system 1 and access the program.

Advantageously, if it is necessary to recover the data related to a username and a password, it is possible to envisage storing such data in a remote archive, for example the archive of a cloud server.

The administrator user can perform a series of actions, for example configuring the program, accessing the system 1 by means of the username and password, changing the language displayed on the interface. The administrator user can make changes to the patient data, perform new examinations for each patient, consult the examinations carried out by each patient and transmit the results of the examinations of the patient, for example by e-mail.

The program also envisages an operator user, who can perform the same actions as the administrator except for the configuration of the program. The program configuration comprises the possibility of calibrating the device for the acquisition of images and to set predetermined parameters of the device such as, by way of example, contrast and brightness. The program configuration also comprises the activation or deactivation of an examination from the examination list in addition to the possibility of enabling and/or disabling the saving of images on a network folder.

The program implemented by the system 1 further comprises instructions which cause the system 1 to perform the step of acquiring data related to a new patient or data related to a patient already registered by means of the user interface 23 of the control unit 5.

In particular, the operator assigned to perform the diagnostic examination or examinations enters the information related to a new patient or selects an existing patient from the stored patient list. A data sheet for each patient is thereby created. The data can be stored in the memory of the system 1 and/or on a remote server 200, for example a cloud server.

The program envisages changing the patient information, when it needs to be updated, or deleting the patient information. In the latter case, the program envisages, in particular, assigning a "deleted" status to the patient without actually deleting the related information in order to store the information in the event of an accidental deletion.

The program then envisages the step of displaying, by means of the user interface 23, a list of examinations which can be carried out by the system 1 (step b).

The program then envisages acquiring information about the examination or examinations which the patient must perform. If the patient must perform more than one examination, it is possible to choose the order in which to perform the examinations. The choice of the order of performance of the examinations is preferably made by means of the user interface 23.

The step of acquiring information about the examination or examinations to be carried out envisages finding such information from the memory of the system 1 and/or from the remote server 200, for example from the cloud server, and possibly displaying it on the user interface 23. Alternatively, the operator can enter, by means of the user interface 23, information regarding the examination or examinations which the patient must carry out and preferably save this information, on the memory of the system 1 and/or on the remote server 200.

Preferably, the program envisages displaying, by means of the user interface 23, information on the emission of a light at a predetermined wavelength to illuminate at least one eye of said patient (step d).

The operator then receives an indication regarding the light to be emitted, by means of the lighting device 12, to illuminate at least one eye of the patient. The light can be, for example, white, red, infrared. The operator then activates the lighting device 12 to emit the light at the indicated wavelength. Alternatively, it is possible to envisage that the program proceeds to automatically activate the lighting device 12, by means of the control unit 5, so as to emit a light at a predetermined wavelength, which depends on the type of examination selected.

Thereafter, at least one image of at least one eye of the patient is acquired (step e).

The step of acquiring at least one image of at least one eye of the patient occurs by means of the image detection and acquisition device 3. Alternatively, the system 1 can use a different image acquisition device, preferably communicating wirelessly with the control unit 5, for example the device associated with the microscope 100.

The next step is to save the at least one acquired image and process it by means of the control unit 5 (step f).

Advantageously, the step of saving the at least one image is carried out in the memory of the system 1 and/or in a remote server 200, for example a cloud server.

The step of processing the image can envisage, for example, modifying the contrast, brightness, controlling the blurring of the image, carrying out three-dimensional processing or identifying the pupil and measuring it, depending on the type of examination which is carried out.

The program then envisages saving the reprocessed image.

The program envisages to automatically repeat the aforesaid steps, in particular steps d-f, until all the selected examinations have been performed. When a patient must perform more than one examination, the program therefore allows to perform the examinations in succession in the same session. This is beneficial to both the patient and the operator, as it results in considerable time savings.

The program can also comprise, for some types of examinations, the step of displaying on the user interface 23 a questionnaire and receiving further data related to the patient which are useful for the diagnosis. The questionnaire is displayed after the acquisition, saving and processing steps of the images of the patient's eyes.

The program further envisages, for specific types of examinations, displaying, always by means of the user interface 23, information related to the use of the diffuser member 19 associated with the first module 2. In essence, the program envisages the step of displaying information on the need or not for the use of the diffuser member 19.

Some examinations which can be selected are reported below.

### Examination examples

The list of examinations which can be carried out by means of the system 1 comprises meibography, interferometry, an examination called "Demodex", an examination called "Redness Efron grading", an examination of the pupil called "Pupil", and an examination of the meniscus called "Meniscus".

The meibography examination is intended to evaluate the level of dysfunction of the meibomian glands. The lipid part of the tear film is provided by the meibomian glands, found in the inner part of the eyelids. The lack of meibomian glands is called "Meibomian Gland Dysfunction" (MGD) and its severity is classified according to the international scale of Dr Heiko Pult. The severity levels range from level 0, where all the glands are present, or the lack of glands is 0%, to level 4 where the lack of glands is comprised between 75% and 100%. The meibography examination allows to acquire an image of the glands and to automatically provide the level of dysfunction according to the Pult scale.

If the meibography examination is selected, the program displays information regarding the emission of an infrared light and the operator proceeds to activate the infrared light.

The operator or, alternatively, the control unit 5, then activates the lighting device 12 to illuminate at least one eye of the patient and at least one image of the patient's eye is acquired by the image detection and acquisition device 3. Thereafter, the at least one acquired image is saved and processed by means of the control unit 5.

The image processing envisages changing the contrast and brightness as well as dividing the image into a series of areas and calculating the percentage of dark pixels for each area so as to display, by means of the user interface, an indication of the image quality based on the percentage of dark pixels. The indication can be, for example, that the image is of low, medium or high quality.

The program also envisages displaying information on the user interface on the possibility of displaying the image in a three-dimensional manner and the operator can choose to carry out a three-dimensional display. The information can consist of an icon indicating the three-dimensional display.

If the aforesaid steps have been performed for only one eye, they are repeated, if necessary, for the other eye of the patient.

Finally, the program preferably envisages the step of displaying a questionnaire on the user interface and receiving further data related to the patient which are useful for diagnosis.

Following the completion of the questionnaire by the operator, the program envisages displaying information on the user interface related to the severity level of the dysfunction.

The "Demodex" examination aims to detect any presence of mites by visually inspecting an image acquired at a high magnification. Demodex is an eight-legged mite belonging to the family Demodecidae, which is naturally found on the faces of human beings since childhood, but especially in adulthood. The presence on the skin increases with age. These mites live in or around hair follicles and sebaceous glands and are part of what is called the cutaneous microbiota or cutaneous microbial flora, i.e., the microorganisms which naturally live on our skin. Throughout their lives, they accumulate waste in their abdomen, which is released only upon their death.

However, these parasites can generate conditions which favour the appearance of certain symptoms and pathologies in cases where they multiply uncontrollably. In particular, Demodex can be the cause of blepharitis.

In the case of the "Demodex" examination, the program comprises the step of acquiring at least one image of the patient's eye, by means of an image acquisition device, preferably the device associated with the microscope 100. Thereby, it is in fact possible to acquire an image at an appropriate magnification by exploiting the magnification capability of the microscope 100. The image is saved and modified by means of the control unit 5. The modified image is saved again.

If it is necessary to acquire at least one image of the patient's other eye, the aforesaid steps are repeated for the other eye.

Subsequently, the program displays the data related to the patient, or the patient card, on the user interface.

The interferometry examination aims to evaluate the thickness of the surface lipid layer which is above the normal tear film in addition to the thickness of the fluid layer covering the front surface of a contact lens. In general, such thickness estimates are based on the colour and shape of the fringes which are generated as interference reflective structures. The Keeler^{©} Tearscope Atlas is used as a reference.

If the interferometry examination is selected, the program displays information regarding the emission of a white light and the need to use a diffuser member 19. The operator then proceeds to activate the white light and associate the diffuser member 19 with the first module 2. In this case as well, the activation of the white light can alternatively be carried out by the control unit 5.

The program then envisages acquiring at least one image of an eye of the patient, saving the acquired image and processing it by means of the control unit 5. The image processing comprises, for example, the steps of changing the brightness and/or contrast, of verifying the blurring of the image. The reprocessed image is saved again.

If it is necessary to acquire at least one image of the patient's other eye, the aforesaid steps are repeated for the other eye. Subsequently, the program displays the data related to the patient, or the patient card, on the user interface.

The purpose of the "Redness Efron grading" examination is to evaluate the percentage of redness, due to blood capillaries, and the severity of redness in the conjunctival area. It must be considered that the pathology of dry eye has an inflammatory component and redness is a symptom of inflammation. Therefore, monitoring redness in patients with dry eye is a valuable aid in order to diagnose the conditions, severity and recovery after treatments.

When the "Redness Efron grading" examination is selected, the program displays information regarding the emission of a white light and a white light is activated by an operator or by the control unit 5. Thereafter, the program envisages acquiring at least one image of at least one eye of the patient and saving the acquired image.

Subsequently the image is processed, for example the brightness and/or contrast are changed. Furthermore, the image processing consists of dividing the image into a series of areas and calculating the ratio of the light pixels to the dark pixels for each area. It is also possible to process the image three-dimensionally.

The program envisages displaying values indicative of the state of health of the patient's eye on the user interface and receiving data on the selected indicative value. Such a value, together with the percentage of black pixels with respect to white pixels previously calculated, are saved as data indicative of the result of the examination.

If it is necessary to acquire at least one image of the patient's other eye, the aforesaid steps are repeated for the other eye. The program then displays patient-related data on the user interface.

The "Pupil" examination aims to provide information regarding the diameter of the pupil, in a condition of ambient light. In the "Pupil" examination, the program shows information regarding the emission of light in the infrared range and the need to remove the diffuser member, where present. Next, the infrared light is activated by an operator or by the control unit 5. Thereafter, the program envisages acquiring at least one image of at least one eye of the patient and saving the acquired image.

Subsequently the image is processed, e.g., the brightness and/or contrast are changed, the pupil is identified and a pupil size measurement is performed.

The reprocessed image is saved and, if it is necessary to acquire at least one image of the patient's other eye, the aforesaid steps are repeated for the other eye. Finally, the program displays the data related to the patient on the user interface.

The "Meniscus" examination is intended to investigate the amount of accumulated tears which is used as an index of ocular dryness. The examination allows to obtain an infrared image of the patient's eye showing, in particular, a lower part of the eye in which tears generally accumulate.

With regard to the "Meniscus" examination, the program envisages acquiring at least one image of at least one eye of the patient and saving the acquired image.

Then the image is modified and the image is saved as modified. In particular, the step of modifying the image envisages calculating at least one distance between two points of interest.

If it is necessary to acquire at least one image of the patient's other eye, the aforesaid steps are repeated for the other eye. Finally, the program displays the data related to the patient on the user interface.

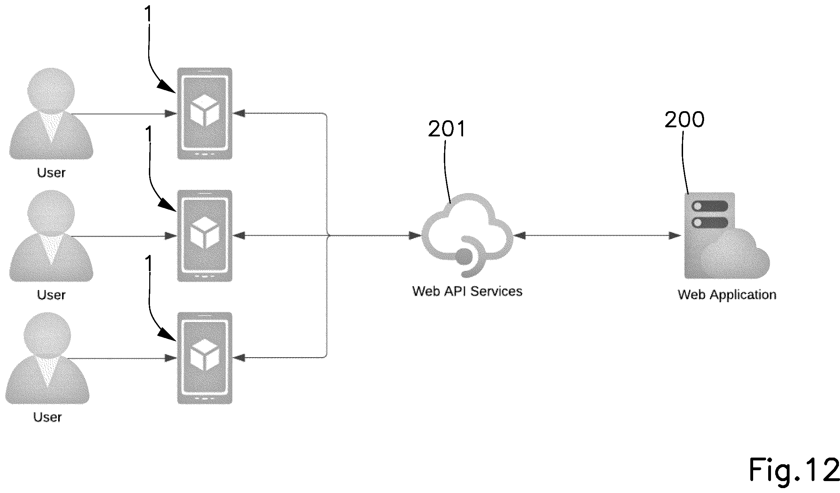

A further aspect of the present invention relates to a data transmission system comprising one or more modular systems 1, each implementing the above-described program (see Figure 12). The systems 1 transmit data, by means of the internet, to a remote server 200 and receive data from the remote server by means of a web API (Application Programming Interface) 201. The web API works as an interface between the applications, i.e., between the application installed in each system 1 and the application related to the server (see Figure 12).

It is possible to envisage using a cloud server.

The remote server 200 comprise means for receiving data from at least one modular system 1, means for processing the data, and means for transmitting further data to the system 1.

The aforesaid data transmission system allows to carry out a remote configuration of the program installed on each system 1, in a differentiated manner for each customer. The server 200 transmits, by the relative transmission means, data to each modular system 1 regarding the activation and/or deactivation of an examination from the examination list, on the settings of the at least one image acquisition device used in the examinations and on the calibration of the same device. The server 200 transmits information to each system 1 about enabling and/or disabling the function of saving files to a data archive of the server 200.

The server 200 transmits activation code data to the systems 1 so as to enable the activation of each program implemented by a related modular system 1.

The modular systems 1 transmit to the server 200, by their transmission means, data related to the patients, the images acquired for each patient and information on the outcome of the examination or examinations carried out by each patient.

The operation of the system can easily be understood from the preceding disclosure.

Initially the system 1 is arranged inside a transportable container, in which some components can be stored in disassembled condition, for example the control unit 5 and the microscope 100.

At the time of use, the operator proceeds to mount the non-assembled components and to mount the system 1, by means the coupling means 10, onto a base body of installed equipment, for example at a medical centre. Alternatively, if manual use of the system is envisaged, the operator proceeds to grasp the resting and/or gripping element 7.

The operator then proceeds to orient the image detection device 3 towards the patient and to orient the control unit 5 towards him/herself so as to correctly display the user interface 23.

The operator then proceeds to activate the computer program disclosed above so as to perform predetermined diagnostic examinations on the patient by means of the system 1. If more than one diagnostic examination must be performed, the system 1 performs the examinations in succession until all the examinations are performed.

At the end of the diagnostic examination or examinations, the operator can store the modular system for new use.

The modular system according to the present invention achieves the object of performing diagnostic procedures in a simple and effective manner thanks to the compact structure of the system which makes it easier for the operator to use and handle. The compact structure is due to the configuration of the support member and, in particular, to the elongated shape of the resting and/or gripping element extending on a vertical plane, preferably along a vertical direction, and to the provision of a seat for the first module at one end of such an element and a seat for the second module on the resting and/or gripping element itself. This configuration further contributes to reducing the system footprint.

In addition, the operating portion defining a seat for the first module therein further contributes to making the system compact.

Furthermore, the simplification in the performance of diagnostic procedures is also due to the computer program implemented by the system which allows an operator to be effectively guided on how to carry out a specific examination. In particular, the program acquires information about the patient and the examination or examinations to be carried out and, based on such information, acquires at least one image of the patient's eye and processes it appropriately.

A prerogative of the present invention is the fact of having devised a program which allows the examinations to be performed in succession within the same session. This reduces the time required to carry out a diagnosis for each patient.

Finally, it is observed that the configuration of the support member provided with a region with a predetermined radius of curvature is ergonomic.

The system described by way of example is susceptible to numerous modifications and variations according to different needs.

In the practical implementation of the invention, the materials employed, as well as the shape and sizes, may be any according to requirements.

Where technical features mentioned in each claim are followed by reference marks, such reference marks have been included for the sole purpose of increasing the understanding of the claims and consequently they do not have any limiting value on the scope of each element identified by way of example by such reference marks.

## Claims

1. A portable modular system for diagnostic instruments comprising a first module (2) comprising at least one image detection and acquisition device (3), for example a camera or a video camera, and a second module (4) comprising a control unit (5) configured to receive and process the images acquired by said image detection and acquisition device (3), said control unit (5) being provided with a user interface (23); a support member (6) configured to removably support said first module (2) and said second module (4) in respective positions, **characterized in that** said control unit comprises an electronic processor and a memory readable by said electronic processor comprising instructions which when performed by said electronic processor, cause the electronic processor to perform the following steps:
a. acquire information related to a new patient or related to a patient already registered by means of said user interface (23) of said control unit (5);
b. display, by means of said user interface (23), a list of examinations which can be performed by the system (1);
c. acquire information about the examination or examinations which the patient must perform;
d. preferably display, by means of said user interface (23), information on the emission of a light at a predetermined wavelength to illuminate at least one eye of said patient;
e. acquire at least one image of at least one of the patient's eyes;
f. save said at least one image and process it by means of said control unit (5);
g. repeat said steps d-f until the end of the performance of all the selected examinations.

2. The system of claim 1, wherein said support member (6) comprises a resting and/or gripping element (7) having an elongated shape extending, in a configuration of use, on a substantially vertical plane, and an operating portion (8), associated with one end of said resting and/or gripping element (7), which shapes a first seat (9) adapted to house said first module (2), said resting and/or gripping element (7) also shaping a second seat (90) for said second module (4).

3. The system of claim 2, wherein said operating portion (8) defines a cavity therein which is adapted to define said first seat (9) for said first module (2).

4. The system of claim 1 or 2, wherein said second seat (90) is made by a recess, said second module (4) comprising attachment means (21) associated, in a configuration of use, with said recess.

5. The system of claim 2 or 3 or 4, wherein said resting and/or gripping element (7) comprises a first portion (7a), extending along a substantially straight direction, and a second portion (7b), connected to said first portion (7a), extending along an inclined direction with respect to the direction of said first portion (7a), on the same lying plane of said first portion (7a), said first portion (7a) and said second portion (7b) defining a curved region adapted to facilitate gripping said resting and/or gripping element (7).

6. The system of any one of claims 2-5, wherein said resting and/or gripping element (7) has a plurality of grooves (11) made on at least a part of the outer surface, adapted to facilitate gripping by an operator.

7. The system of any one of claims 2-6, wherein said support member (6) is made of a pair of half-casings (6a, 6b) coupled to each other, said first module (2) being housed between said half-casings (6a, 6b) at said operating portion (8).

8. The system of any one of the preceding claims, wherein said first module (2) further comprises a lighting device (12), frontally associated with said image detection and processing device (3), said lighting device (12) comprising a plurality of light emitting diodes (13) and an electric control circuit (14) of said light emitting diodes (13).

9. A computer program comprising instructions which cause the system of any one of the preceding claims to perform the following steps:
a. acquire information related to a new patient or related to a patient already registered by means of said user interface (23) of said control unit (5);
b. display, by means of said user interface (23), a list of examinations which can be performed by the system (1);
c. acquire information about the examination or examinations which the patient must perform;
d. preferably display, by means of said user interface (23), information on the emission of a light at a predetermined wavelength to illuminate at least one eye of said patient;
e. acquire at least one image of at least one of the patient's eyes;
f. save said at least one image and process it by means of said control unit (5);
g. repeat said steps d-f until the end of the performance of all the selected examinations.

10. The program of claim 9, wherein said step g. of repeating said steps d-f until the end of the performance of all the selected examinations is performed automatically.

11. The program of claim 9 or 10, wherein it comprises, before step d., the step of choosing the order of performance of said examinations, if said patient must carry out more than one examination.

12. The program of claim 9 or 10 or 11, wherein said steps d-f are repeated in succession if said patient must carry out more than one examination.

13. The program of any one of claims 9-12, wherein it comprises, following step f., the further step of displaying on said user interface (23) a questionnaire associated with a predetermined diagnostic examination in progress and receiving further data regarding the patient.

14. The program of any one of claims 9-13, wherein it comprises the step of displaying, by means of said user interface (23), information related to the use of a diffuser member associated with said first module (2).

15. The program of any one of claims 9-14, wherein said list of examinations comprises: meibography, interferometry, an examination which has the aim of detecting the possible presence of Demodex mites by visual inspection of an image acquired at a high magnification, an examination which evaluates the percentage of redness and the severity of redness in the conjunctival area, an examination which has the aim of providing information on the pupil diameter in a condition of ambient light, an examination which has the aim of investigating the amount of accumulated tears, in particular by investigating a lower portion of the patient's eye in which tears generally accumulate.
